# EUROPEAN PATENT APPLICATION

(11) **EP 3 735 987 A1**
(43) Date of publication of application: **11.11.2020**
(21) Application number: 18852932.5
(22) Date of filing: 08.09.2018
(51) Int. Cl.: A61K 47/00, A61K 39/395, A61P 35/00

(54) **AMANITIN ANTIBODY CONJUGATE**

(30) Priority: 08.09.2017 CN 201710804207
(71) Applicant: Sichuan Baili Pharm Co. Ltd, Chengdu, Sichuan 611130 (CN)
(72) Inventor: ZHU, Yi, Sichuan 610041 (CN); LI, Jie, Sichuan 610041 (CN); WAN, Weili, Sichuan 610041 (CN); YU, Yongguo, Sichuan 610041 (CN); ZHUO, Shi, Sichuan 610041 (CN)
(74) Representative: Hobson, David James
(86) International application number: PCT/CN2018/104712
(87) International publication number: WO 2019/047941

(57) **Abstract**

A bicyclic octapeptide derivative is conjugated to a corresponding target-binding group by a special chemical structure. The structure of the derivative is stable in blood plasma and decomposes into a drug as an active ingredient in a specific biological environment, thereby maximizing killing effect on target cells and minimizing toxic side effects on non-target cells. The derivative can be used in the treatment of various malignant tumors.

## Description

### Technical Field

The present invention belongs to the field of biopharmaceutical technology, and specifically relates to an amanitin antibody conjugate.

### Background Art

Amanitin is a bicyclic peptide of 8 amino acids which is one of several amanitin toxins isolated from highly toxic mushrooms; there are currently nine natural amanitins which have been isolated and purified: *α*-amanitin, *β-*amanitin, *γ*-amanitin, *ε*-amanitin, amanin, amaninamide, amanullin, amanullinic acid and proamanullin, where *α-*amanitin and *β*-amanitin constitute the primary toxins responsible for causing death. Amanitins are a class of slow-acting toxins that inhibit the transcription of eukaryotic RNA polymerase II and RNA polymerase III, leading to protein loss and cell death. This class of toxins produces extremely high inhibition of RNA polymerase II, with a KD of up to 3 nM, and they are repeatedly absorbed in the body due to entero-hepatic circulation in the gastrointestinal tract, causing severe damage to the organs of the human body such as the liver, kidneys, heart and lungs.

The toxicity of amanitin is greatly reduced or even rendered relatively inert when it is coupled with a large biomolecular carrier (such as an antibody molecule), and only after the biomolecular carrier is removed in a specific physiological environment will amanitin exhibit cytotoxicity.

In recent years, a number of research institutes and companies around the world have structurally modified amanitin molecules to obtain antibody-toxin conjugates of pharmaceutical value, including: Heidelberg Pharma AG (Germany) which has coupled the 1- position, 3-δ-position or 6'-phenol hydroxyl group of natural amanitin to a monoclonal antibody using a linker; Agensys Co. (US) which has structurally modified the phenyl on the indole ring of *α*-amanitin to link it to an antibody from the 6'-phenol hydroxyl or 7'- position using a linker; Hangzhou Duoxi Biotechnology Co., Ltd. which has performed nitration of the 5'- and 7'-positions of an amanitin derivative to couple it to an antibody using a linker with an amide structure; Leg °Chem Biosciences Co. (Korea) which has also studied methods for linking to antibodies using a linker from the 6'-phenol hydroxyl group as well as 3-δ- position. In the above synthesis methods, the method used to splice the linker from the 6'-phenol hydroxyl group requires multi-step protection and deprotection of hydroxyl groups located at other sites of the amanitin molecule, and the corresponding synthesis route is complicated; additionally, uncertain positioning in the substitution reaction performed on the 5'- and 7'- positions of the benzene ring readily leads to difficulty in product separation and low yield.

### Summary of the Invention

Based on the above prior art, the object of the present invention is to provide a conjugate for a bicyclic octapeptide amanitin derivative and a biomolecule that is stable within the circulatory system and is cleaved after endocytosis by the target cell, releasing an amanitin derivative that acts as an RNA polymerase inhibitor, producing a high level of toxicity to the cell through specific inhibition of eukaryotic mRNA synthesis.

In order to achieve the above object, the present invention employs the following technical solution: A toxin conjugate as claimed in Structural Formula (I) or a pharmaceutically acceptable salt thereof: 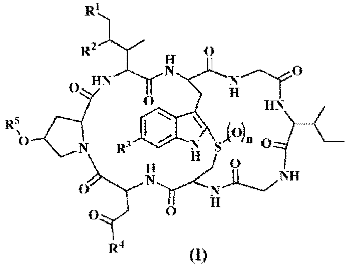 Wherein:
n = 0, 1 or 2,
R¹ corresponds to -H or -OH,
R² corresponds to -H or -OH,
R³ corresponds to -H, -OH or C₁₋₆ alkyl,
R⁴ corresponds to -NH₂ or -OH,
R⁵ corresponds to -L-A,
A corresponds to the biological macromolecule moeity that binds to a target, and
L corresponds to any chemical structure connecting an amanitin derivative and a biological macromolecule. Preferably, the chemical structure of L includes a cleavable or non-cleavable structure.

Preferably, A includes an antibody or antigen-binding fragment or antigen-binding polypeptide thereof. Preferably, the conjugation site of L and the target-binding biomolecule A includes the following structure:

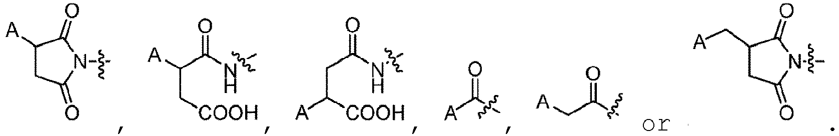

Preferably, L is connected to the toxin via an ester or ether.

The present invention also includes a pharmaceutical composition containing the aforementioned toxin conjugate or a pharmaceutically acceptable salt thereof as an active ingredient.

The present invention also includes a use of the aforementioned toxin conjugate or a pharmaceutically acceptable salt thereof in the preparation of an anti-tumor drug or anti-cancer drug.

Furthermore, said antitumor drug or anti-cancer drug corresponds to an anti-lung cancer drug, an anti-kidney cancer drug, an anti-urinary tract cancer drug, anti-colorectal cancer drug, anti-prostate cancer drug, anti-glioblastoma drug, anti-ovarian cancer drug, anti-pancreatic cancer drug, anti-breast cancer drug, anti-melanoma drug, anti-liver cancer drug, anti-bladder cancer drug, anti-malignant lymphoma drug, anti-leukemia drug, anti-gastric cancer drug or an anti-esophageal cancer drug.

Definitions: In accordance with standard practices in the field, the 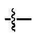 symbol used in formulas and tables pertaining to the present invention represents a bond at a core or nuclear junction of a moeity or substituent with the compound structure.

In accordance with standard practices in the field, in the context of the present invention hetero (atom, alkyl, aryl, cyclic group) refers to a corresponding chemical structure containing an atom other than a carbon atom.

### Abbreviations and Symbols:

Boc: tert-butoxycarbonyl;
PyBOP: Benzotriazole-1-yl-oxytripyrrolidinophosphonium hexafluorophosphate;
Cit: L-Citrulline;
CO₂: Carbon dioxide;
DCM: Dichloromethane;
DIPEA: *N,N*-Diisopropylethylamine;
DMF: *N,N*-Dimethylformamide;
DMSO: Dimethyl sulfoxide;
DPBS: Dulbecco's phosphate buffer saline;
DTPA: Diethylenetriaminepentaacetic acid;
DTT: *DL*-Dithiothreitol;
EA: Ethyl acetate;
EDTA: Ethylenediaminetetraacetic acid;
FBS: Fetal bovine serum;
HATU: (1-[Bis(dimethylamino)methylene]-1H-1,2,3-triazolo[4,5-b]pyridinium 3-oxide hexafluorophosphate; H₂O: Water;
HOBt: 1-Hydroxybenzotriazole;
mAb: Monoclonal antibody;
MEM: Minimum essential medium;
MTS: 3-(4,5-Dimethylthiazol-2-yl)-5-(3-carboxymethyl)-2-(4-sulfophenyl)-2H-tetrazole, internal salt
MTT: 3-(4,5-Dimethylthiazolyl-2)-2,5-diphenyl tetrazolium bromide salt
PAB: p-Aminobenzyloxy;
PBS: Phosphate buffered saline;
Sodium Pyruvate: Sodium pyruvate;
THF: Tetrahydrofuran;
TLC: Thin layer chromatography;
Tris: Tris(hydroxymethyl)aminomethane;
Val: Valine;
Trt-Cl: Chlorotriphenylmethane;
TBTU: *N,N,N',N*'-Tetramethyl-O-(benzotriazol-1-yl)uronium tetrafluoroborate;
HOBT: 1-Hydroxybenzotriazole;
TFA: Trifluoroacetic acid;
TBS-Cl: *t*-Butyldimethylsilyl chloride;
HOSu: *N*-Hydroxysuccinimide;
Na₂CO₃: Sodium carbonate;
EDCI: *N*-Ethyl-*N*'-(3-dimethylaminopropyl)carbodiimide hydrochloride

The merits of the present invention are as follows:
In the present invention, we conjugated an antibody molecule with the 2-proline hydroxyl group of amanitin or a derivative molecule thereof using a pharmaceutically acceptable linking structure, and were thereby able to obtain a conjugate that is stable and non-toxic in plasma but can be cleaved to release toxic molecules in diseased cells, and were pleasantly surprised to find that said linking method is simple and efficient and greatly satisfies a need for the industrial-scale mass production of such products.

The present invention discloses a bicyclic octapeptide derivative, which is conjugated with a corresponding target binding group via a specific chemical structure that is stable in plasma and can be cleaved to release an active drug in a specific biological environment to maximize lethality to target cells and minimize toxic side effects to non-target cells, and which can be used in the treatment of a variety of malignant tumors.

### Description of the Figures

Figure 1 shows experimental results obtained from cell line BT474 in Example 13.
Figure 2 shows experimental results obtained from cell line SKBR3 in Example 13.
Figure 3 shows experimental results obtained from cell line N87 in Example 13.

### Specific Embodiments

The present invention is described in further detail below.

### Example 1: Synthesis of Small Molecule Payload ama-1

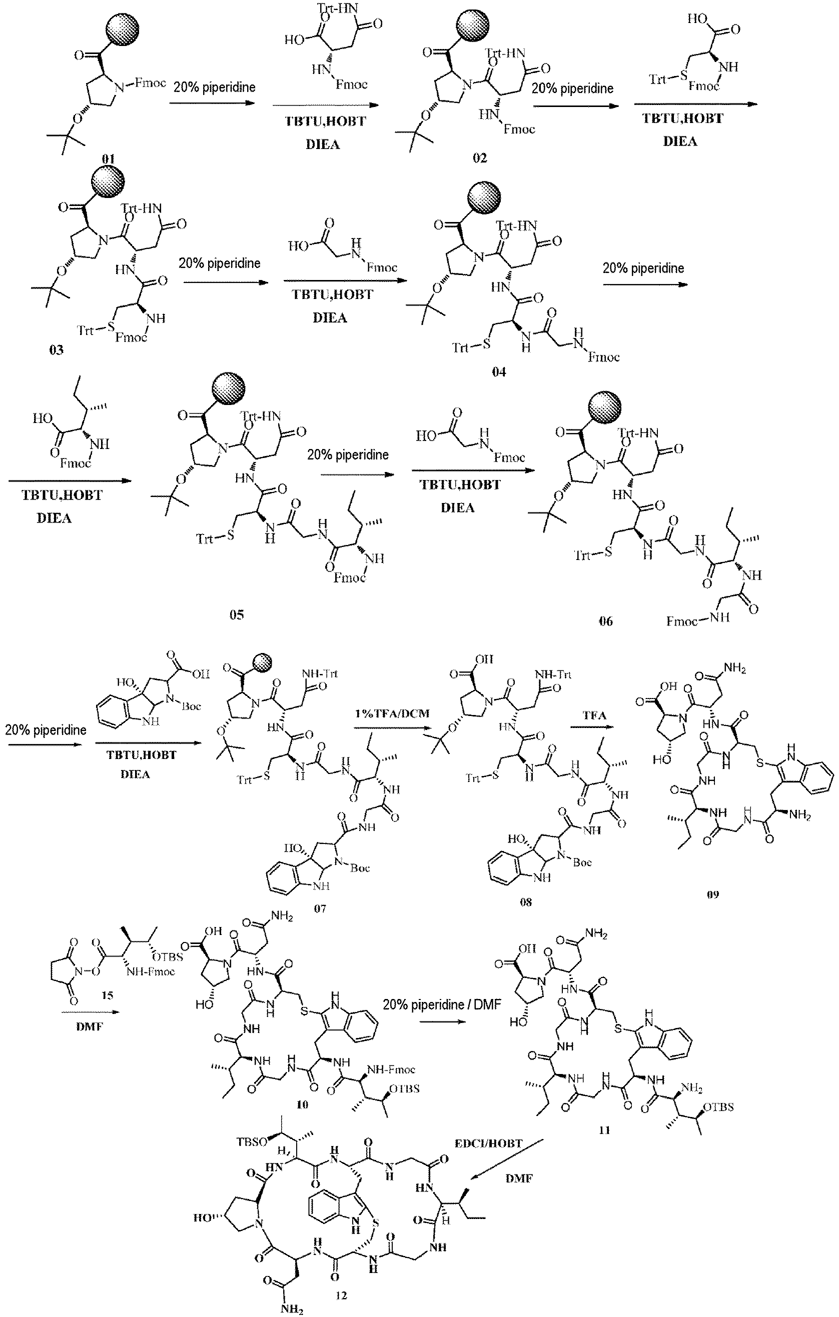

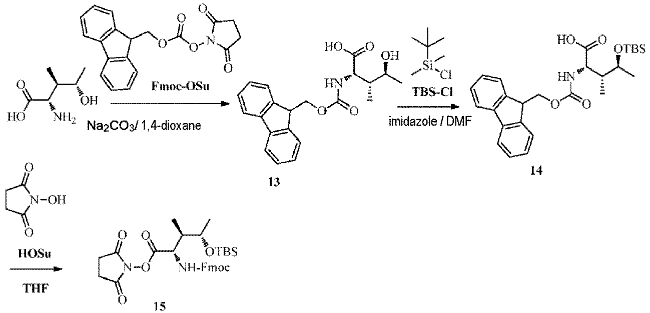

### Solid Phase Synthesis of Intermediate 08:

Using N-fluorenylmethoxycarbonyl-O-*tert*-butyl-L-hydroxyproline preloaded resin as a starting material, the Fmoc protective group was removed with 20% piperidine (20 ml of 20% piperidine in 1 g of resin), after which DMF was added as a solution (20 ml/g), followed by the sequential addition of Fmoc-*N*-trityl-L-asparagine (Fmoc-Asn(Trt)-OH) (3 eq), TBTU (2.5 eq), HOBT (1.8 eq) and DIPEA (6 eq); after the reaction was allowed to proceed at room temperature (28°C) for two hours, washing was performed with DMF (20 ml DMF per 1 g resin each time) three times, after which subsequent amino acids were connected according to the previous procedure; once the final connection was complete, 1% TFA (20 ml per 1 g resin each time, 1% TFA 5 min, repeated three times) was used to perform cleaving from the resin and the solution was spun off, after which stirring was performed with methyl *tert*-butyl ether to induce crystal separation, yielding Compound **08,** with a yield of approximately 54% and a purity of 76%. MS: [M+H]⁺ 1417.6123.

### Synthesis of Compound 09:

10 g of Compound **08** was dissolved with TFA (10 ml/g), after which stirring was performed at room temperature for five hours followed by removal of the TFA under reduced pressure at 50°C, and purification by prep-HPLC, thereby obtaining approximately 4.3 g of pure product, with a yield of 43% and a purity of 95.6%. MS: [M+H]⁺ 760.2144.

### Synthesis of Compound 13:

2.94 g of (*4S*)-hydroxyisoleucine, 40 ml of 1,4-dioxane and ml of saturated sodium carbonate solution were added to a 250 ml single-neck flask and stirred well, after which Fmoc-OSu was added in batches; 10 minutes thereafter, the reaction was continued at room temperature for 12 hours under stirring to complete the raw starting material reaction; 50 ml of water was then added into the reaction solution and the pH was adjusted to approximately 4 using a 5% citric acid solution; ethyl acetate extraction was performed three times (50 ml each time) and the organic layer was collected and washed once with 50 ml of saturated brine, dried with anhydrous sodium sulfate, and concentrated to obtain a pale yellow oily substance. No purification was required and the substance obtained was fed directly into the next step. Yield was >100%.

### Synthesis of Compound 14:

After dissolving the crude product constituted by Compound **13** in 40 ml of DMF, 2.68 g imidazole (2 eq) was added followed by the addition of TBS-Cl in batches; once addition was complete, stirring was performed at room temperature for 12 hours until the raw starting material was completely reacted, after which 50 ml of water and 50 ml of ethyl acetate were added and stirring was performed; the organic layer was then separated, followed by extraction of the aqueous layer twice with ethyl acetate (50 ml each time), and the organic layer was collected, dried with anhydrous sodium sulfate, filtered and concentrated to obtain a light-yellow oily substance which was subject to silica gel column chromatography (eluent: PE:EA = 5:1), thus obtaining 4.5 g of an oily substance. The corresponding two-step yield was approximately 46.6%.

### Synthesis of Compound 15:

Compound **14,** HOSu (1.23 g, 1.15 eq), DCC (2.23 g, 1.15 eq) and 50 ml of THF were added to a 250 ml single-neck flask and stirring was performed at room temperature for six hours under nitrogen gas, next, 50 ml of water and 50 ml of ethyl acetate were added and the mixture was stirred for 10 min, followed by separation of the organic layer and extraction of the aqueous layer twice with ethyl acetate using 50 ml each time, the organic layer was then combined and dried with anhydrous sodium sulfate, filtered, and concentrated to obtain a light-yellow oily substance which was then purified via prep-HPLC to obtain approximately 3.24 g of a white foamy solid, with a yield of 60%. 1H-NMR(400MHz, DMSO-d6): 0.08(s, 6H), 0.86(s, 9H), 0.98(d, 3H, J=8.0Hz), 1.06(d, 3H, *J*=5.6), 1.95(t, *J*=10.8), 2.83(s, 4H), 4.21(dd, 1H, *J*=16.8Hz, 8.0Hz), 4.34(dd, 1H, *J*=12Hz, 4Hz), 4.67-4.73(m, 1H), 7.31(d, 2H, *J*=8.0Hz), 7.34-7.46(m, 2H), 7.70-7.76(m, 2H), 7.89(t, 2H, *J*=12.0), 8.24(d, 1H, *J*= 8.8Hz) ; MS: [M+H]⁺581.34.

### Synthesis of Compound 10

After dissolving 180 mg of Compound **09** using 1.5 ml of DMF, Compound **15** (825 mg, 6 eq) and DIPEA (0.25 ml, 6 eq) were added under nitrogen gas and the reaction was allowed to proceed at room temperature for five hours while monitoring was performed using HPLC. The resulting product was used directly in the next step with no need for further post-processing.

### Synthesis of Compound 11

0.3 ml of piperidine was added to the above reaction solution and the reaction was continued at room temperature under stirring for two hours, after which the reaction was stopped, purified using prep-HPLC (neutral, acetonitrile/pure water system), and target peaks were collected followed by the removal of acetonitrile under reduced pressure, subsequently, lyophilization was performed to obtain 136 mg of a white powdery solid, with a corresponding two-step yield of approximately 52%; MS: [M+H]⁺1003.5654.

### Synthesis of Compound 12

After dissolving 136 mg of Compound **11** with dry DMF, EDCI (130 mg, 5 eq), HOBT (367 mg, 20 eq) and DIPEA (0.12 ml, 5 eq) were added and stirring was performed at room temperature for four hours, after which HPLC was used to confirm reaction completion, next, prep-HPLC purification (neutral, acetonitrile/pure water system) was carried out and target peaks were collected followed by the removal of acetonitrile under reduced pressure, lyophilization was performed to obtain 60 mg of a white powdery solid, with a yield of approximately 45%; MS: [M+H]⁺985.5421.

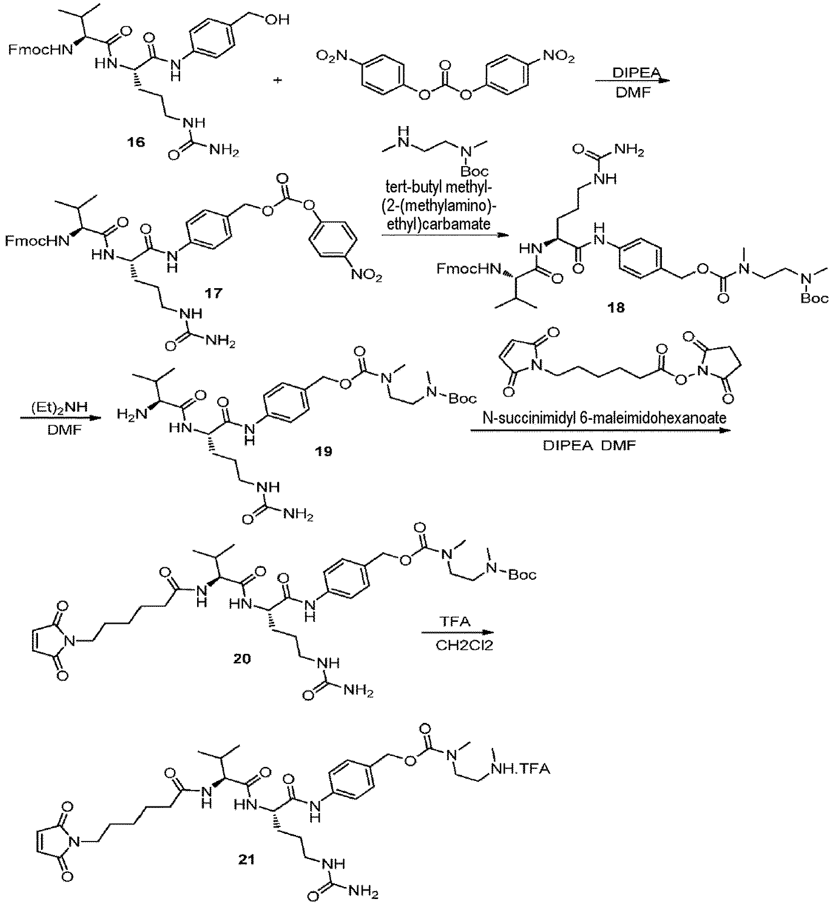

### Synthesis of Compound 17

30 g of Compound **16** (49.92 mmol, 1.0 eq) was added to a 500 ml round bottom flask and dissolved under stirring with 240 ml of DMF, after which 22.76 g of bis(p-nitrophenyl) carbonate (74.87 mmol, 1.5 eq) was added and 9.68 g of DIPEA (74.87 mmol, 1.5 eq) was added dropwise; stirring was then performed at room temperature and the reaction was allowed to proceed for 1.5 hours, with TLC used to confirm that the raw starting materials were completely reacted, and the reaction was then stopped. Post-Processing: 1.5 L of isopropyl ether was added to the reaction flask under vigorous stirring, and stirring was continued for two hours before removing the upper liquid layer, after which 800 ml of isopropyl ether was added to the residue and stirring was continued for one hour, followed by vacuum filtration, the resulting filter cake was then added to 600 ml of isopropyl ether and left to stand overnight while stirring was performed, after which vacuum filtration was performed to obtain 31.2 g of a brownish yellow powdery solid, with a yield of 81.6%. MS: [M+H]⁺767.62.

### Synthesis of Compound 18

31.2 g of Compound **17** (40.73 mmol, 1.0 eq) was added to a 500 ml single-neck flask and dissolved under stirring with 150 ml of DMF, after which 8.1 g of tert-butyl methyl(2-(methylamino)ethyl)carbamate (36.66 mmol, 1.1 eq) dissolved in 50 ml of DMF was added, and the reaction was allowed to proceed at room temperature under stirring for 3.5 hours, TLC was used to confirm that Compound **17** was completely reacted and the reaction was stopped. Post-Processing: 2 L of isopropyl ether was added into the reaction solution and stirring was performed to precipitate out a viscous oily material onto the flask's inner wall, after which the supernatant was poured off, 1 L of isopropyl ether was added, and vigorous stirring was performed; the supernatant was then poured off again, 500 ml of isopropyl ether was added and the mixture was allowed to stand overnight while stirring was performed; finally, vacuum filtration was performed to obtain 27.2 g of a brownish yellow powdery solid, with a yield of 81.9%. MS: [M+H]⁺ 816.73.

### Synthesis of Compound 19

20 g of Compound **18** (24.53 mmol) was weighed out into a 500 ml round bottom flask and 100 ml of DMF was added to the flask to dissolve the compound, 20 ml of diethylamine was then added dropwise in an ice bath for 10 minutes, after which stirring was performed and the reaction was allowed to proceed for two hours at room temperature, with TLC used to confirm that Compound **18** was completely reacted, and the reaction was then stopped. Post-Processing: DMF and diethylamine were removed under reduced pressure, and the resulting product was directly fed into the next step without performing purification.

### Synthesis of Compound 20

In a 500 ml round bottom flask, 150 ml of DMF was added to dissolve the crude product constituted by Compound **19** obtained in the previous step, followed by the addition of 11.7 g of *N*-succinimidyl 6-maleimidohexanoate (37.98 mmol, 1.55 eq) and then 8.4 ml of DIEA (50.63 mmol, 2.06 eq), which was added dropwise, and stirring was performed at room temperature while the reaction was allowed to continue for two hours, with TLC used to confirm that the raw starting material Compound **19** was completely reacted, and the reaction was then stopped. Post-Processing: 1.5 L of isopropyl ether was added into the reaction solution and stirring was performed to precipitate out crystals, resulting in the precipitation of a solid as well as an oily viscous material which stuck to the wall of the flask, the supernatant was decanted and 500 ml of methyl *tert* ether and 500 ml of isopropyl ether were each added to perform stir-washing once, followed by vacuum filtration, the resulting filter cake was dried under reduced pressure at 45°C in a vacuum dryer to obtain 20 g of a crude product in the form of a brownish yellow powdery solid. MS: [M+H]⁺ 787.63.

### Synthesis of Compound 21

0.4 g of Compound **20** was dissolved in 2 ml of 50% TFA dichloromethane solution, after which stirring was performed at room temperature for two hours and TLC was used to confirm the reaction was completed, subsequently, the solution was removed under reduced pressure for further use, and the product was directly fed into the next step, with no need for purification.

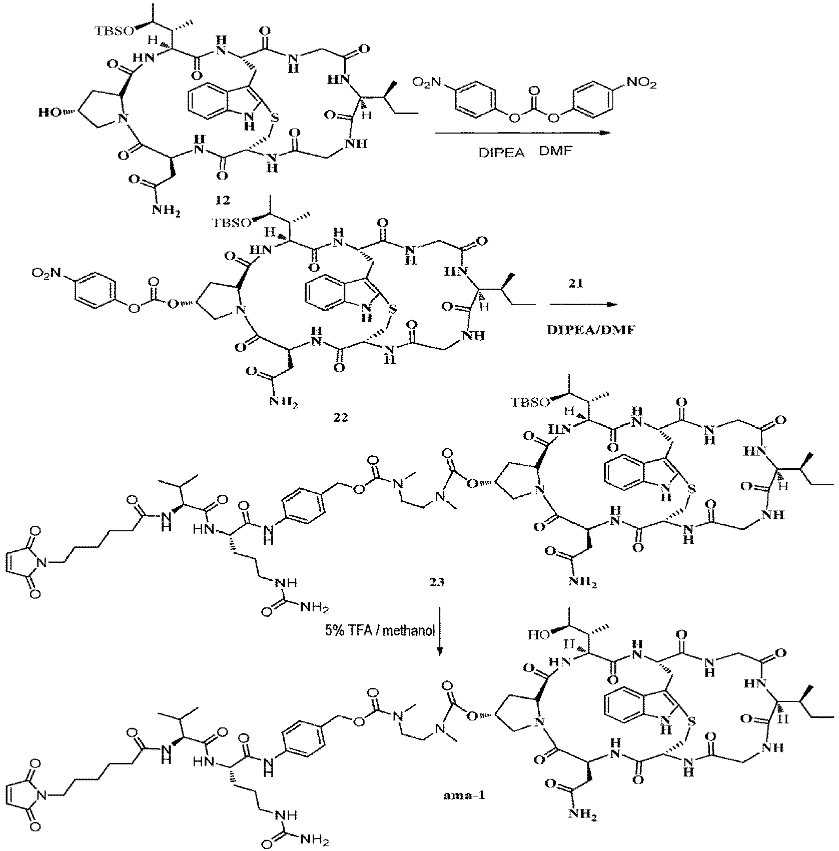

### Synthesis of Compound 22:

100 mg (0.10 mmol, 1.0 eq) of Compound **12**, 304 mg (1.0 mmol, 10 eq) of bis(p-nitrophenyl) carbonate and 3 mL of DMF were added into a 20 mL brown flask and once dissolution was complete, DIPEA (260 mg, 20 eq) was added and the temperature was raised to 28°C for 24 hours under nitrogen gas, with HPLC used to monitor the reaction and ensure that raw material **012** and NPC were completely reacted, the resulting crude product did not need to be processed and was directly fed into the next reaction.

### Synthesis of Compound 23

Crude Compound **21** was added into the reaction solution of Compound **22,** after which a suitable amount of DIPEA was added, maintaining a reaction solution pH ranging from 8 to 9, stirring was performed at room temperature for three hours under nitrogen gas, after the reaction was complete monitored by HPLC, the target product was purified via prep-HPLC to obtain 30 mg of a light-yellow solid. MS: [M+H]⁺ 1697.9150.

### Synthesis of Compound ama-1

15 mg of Compound **23** was added into a 4 mL brown flask, followed by the addition of 1 ml of 5% TFA/MeOH to completely dissolve the compound, the reaction temperature was then raised to 28°C for one hour under nitrogen gas and HPLC (pure water) was used to monitor the reaction, once raw material **23** was completely reacted, the solvent was dried off with nitrogen and HPLC was used to prepare a product with an acetonitrile concentration of approximately 35% which was then lyophilized to obtain 11 mg of a white solid, with a yield (Y) of 42%. MS: [M+H]⁺ 1583.7532.

### Example 2: Synthesis of Small Molecule Payload ama-2

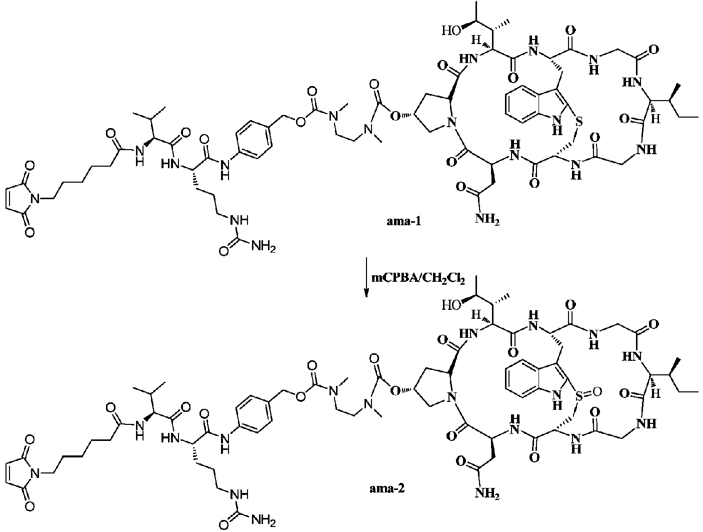

Approximately 6 mg of ama-**1** was dissolved with dichloromethane, followed by the addition of 13 microliters of a dichloromethane solution of 1.0 eq mCPBA (0.05 g/ml), after which stirring was performed at room temperature for two hours and HPLC was used to confirm that the raw starting materials were completely reacted, preparation purification by prep-HPLC was then performed followed by lyophilization to obtain approximately 3 mg of a white powdery solid. The yield was 49.5%. MS: [M+H]⁺1599.8450.

### Example 3: Synthesis of Small Molecule Payload ama-3

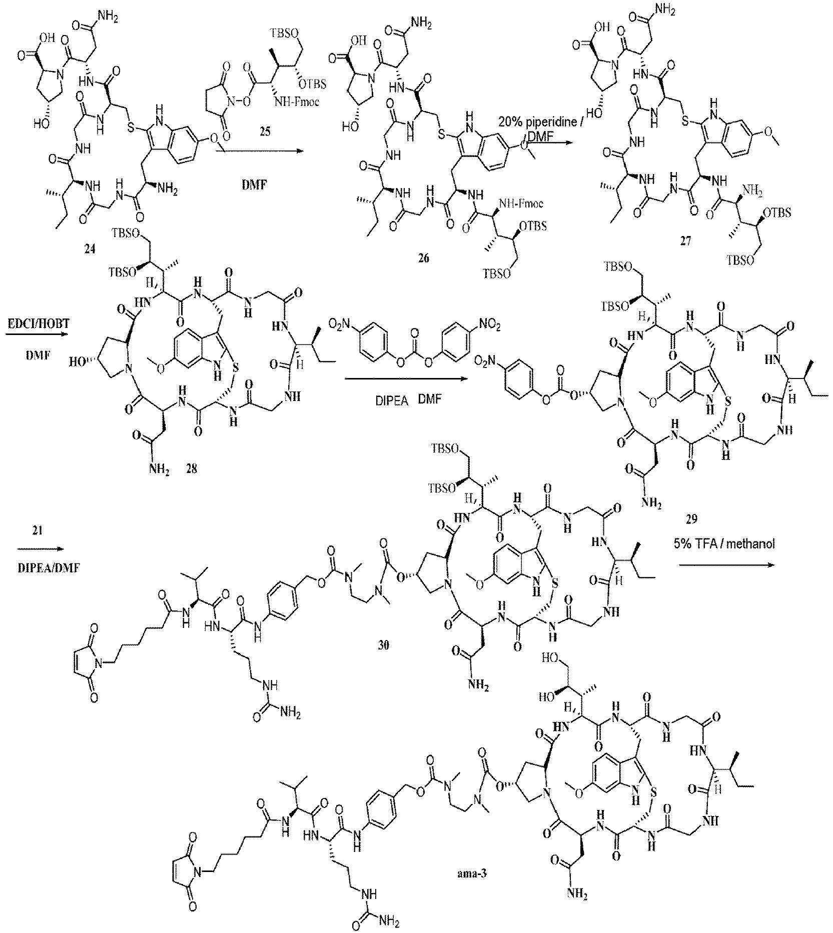

Synthesis of Compound **24:** Referring to the synthesis of Compound 09, approximately 1.2 g of a white solid was obtained. The yield was 25.7%. MS: [M+H]⁺ 790.4254.
Synthesis of Compound **25:** Synthesis was performed with reference to Liang Zhao, et al. Synthesis of a Cytotoxic Amanitin for Biorthogonal Conjugation. Chem Bionchem. 2015, 16, 1420-1425.
Synthesis of Compound **26:** Referring to the synthesis method of Compound **10,** 300 mg of Compound **24** was added, and the product was directly used in the next step without purification.
Synthesis of Compound **27:** Referring to the synthesis method of Compound **11,** purification by prep-HPLC was performed, followed by lyophilization to obtain 195 mg of a white solid, with a total two-step yield of 44.1%. MS: [M+H]⁺ 1163.6341.
Synthesis of Compound **28:** Referring to the synthesis method of Compound **12,** 150 mg of Compound **27** was added to obtain 96 mg of a white solid target compound, with a yield of 65%. MS: [M+H]⁺ 1145.6124.
Synthesis of Compound **29:** Referring to the synthesis of Compound **22,** 80 mg of Compound **28** was added, and the product was directly used in the next step without purification.
Synthesis of Compound **30:** Referring to the synthesis of Compound **23,** following preparation and purification, approximately 59 mg of a light-yellow solid was obtained, with a yield of 45.4%. MS: [M+H]⁺ 1857.9813.
Synthesis of Compound ama-**3**: Referring to the synthesis of Compound ama-**1**, 40 mg of Compound 30 was added and, following preparation and purification, approximately 8.4 mg of a light-yellow solid was obtained. The yield was 24%. MS: [M+H]⁺ 1629.8021.

### Example 4: Synthesis of Small Molecule Payload ama-4

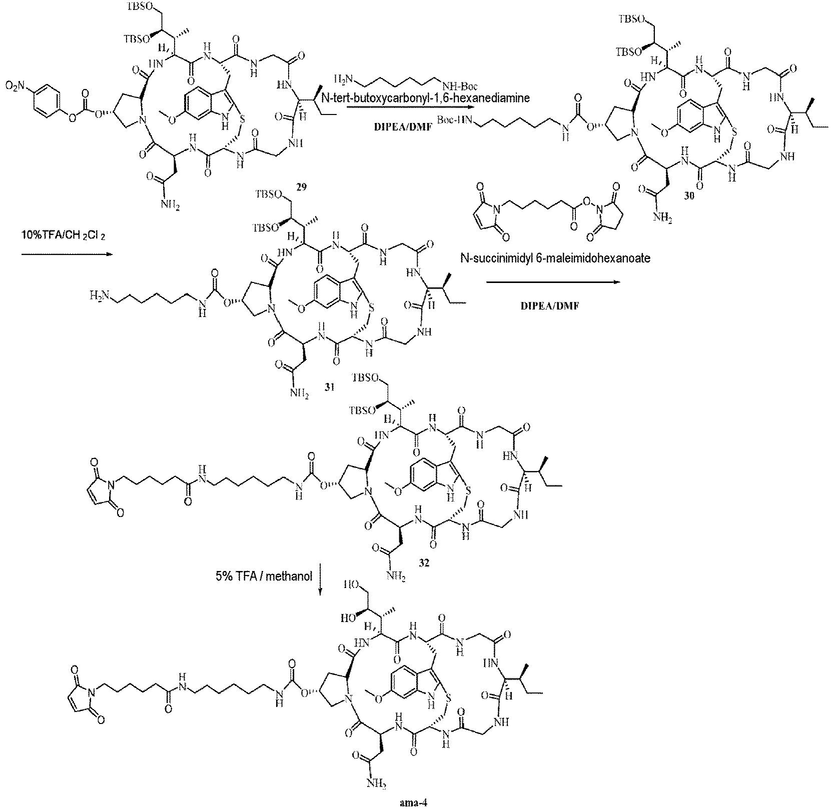

Synthesis of Compound **30:** 49 mg of N-tert-butoxycarbonyl-1,6-hexanediamine (2 eq) was added to the reaction solution of Compound **29** (wherein Compound **28** was replenished to 500 mg, corresponding to the addition in this case of 130 mg, without purification and calculated according to 100% yield) and DIPEA was added dropwise, with the solution pH maintained to within a range of 8 to 9 and stirring was performed at room temperature under nitrogen gas for four hours, after confirming via HPLC that Compound 29 was completely reacted, the reaction was purified by prep-HPLC, target peaks were collected and the organic solvent was spun off, followed by lyophilization to obtain approximately 83.4 mg of a white solid, with a yield of 53%. [M+H]⁺1387.7541.
Synthesis of Compound **31:** 80 mg of Compound **30** was dissolved in 1 ml of a 20% TFA dichloromethane solution and stirring was performed at room temperature for two hours under nitrogen gas, HPLC was used to confirm that Compound 30 was completely reacted, after which the organic solvent was spun off under reduced pressure and the resulting product was set aside for later use.
Synthesis of Compound **32:** After dissolving the crude product corresponding to Compound **31** with 2 mL of DMF, pH was adjusted to 8 to 9 with DIPEA, after which 38 mg (2 eq) of N-succinimidyl 6-maleimidohexanoate was added and stirring was performed at room temperature for six hours under nitrogen gas, after confirming via HPLC that Compound **31** was reacted completely, the reaction was purified by prep-HPLC, target peaks were collected and the organic solvent was spun off, followed by lyophilization to obtain approximately 46 mg of a white solid with a yield of 54%; [M+H]⁺1480.7841.
Synthesis of Compound ama-**4**: Referring to the synthesis of Compound ama-**3**, 44 mg of Compound **32** was added and lyophilization was performed to obtain 21 mg of target compound, with a yield of 56.4%; [M+H]⁺1152.5431.

### Example 5: Synthesis of Small Molecule Payload ama-5

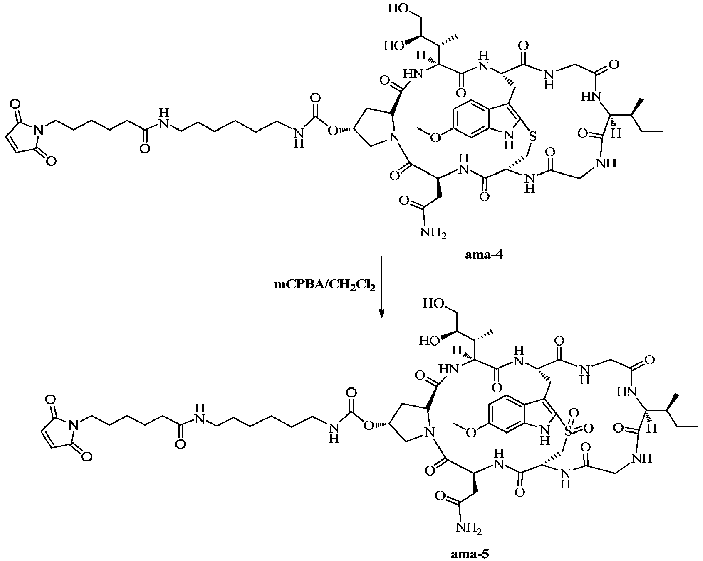

12 mg of ama-**4** was dissolved with dichloromethane, followed by the addition of 165 microliters of a dichloromethane solution of 5.0 eq mCPBA (0.05 g/ml), after which stirring was performed at room temperature for two hours and HPLC was used to confirm that the raw starting materials were completely reacted, preparation purification by prep-HPLC was then performed followed by lyophilization to obtain approximately 4 mg of a yellow powdery solid. The yield was 32.9%. MS: [M+H]⁺1268.5821.

### Example 6: Synthesis of Small Molecule Payload ama-6

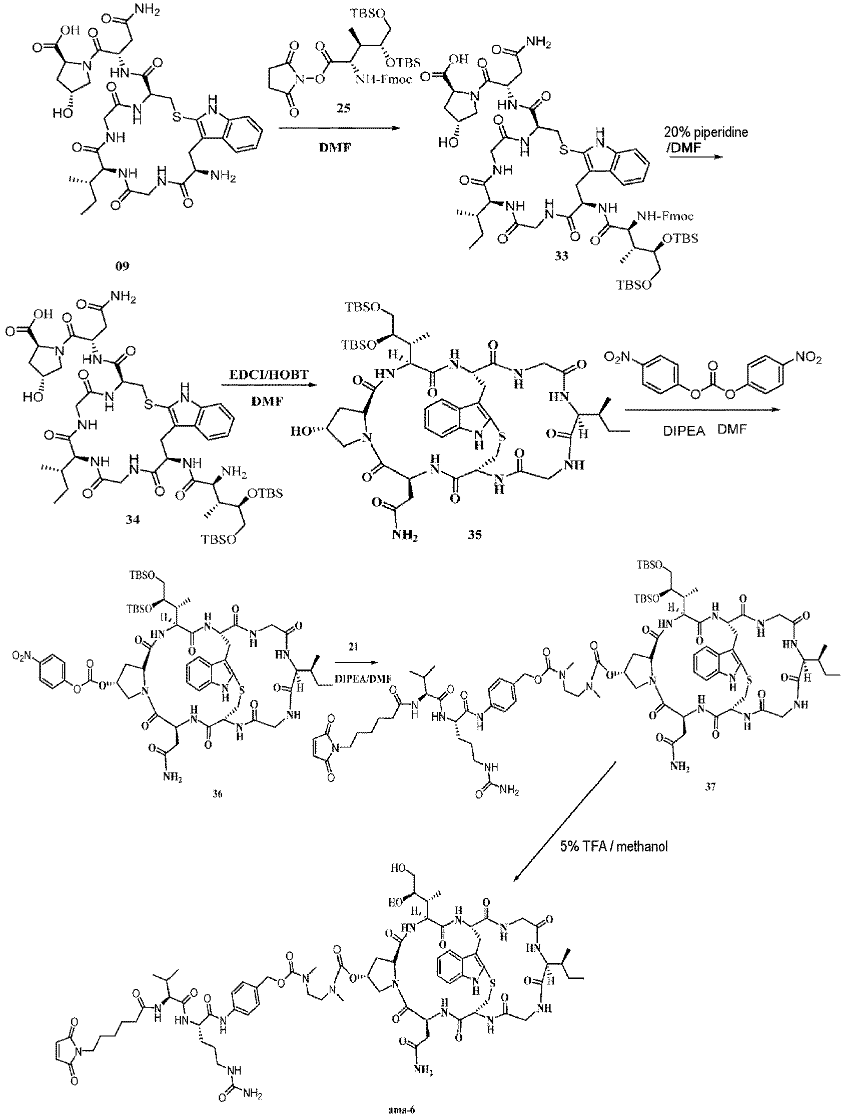

Synthesis of Compound **33:** Referring to the synthesis of Compound **10,** 300 mg of Compound **09** was added, and the product was directly used in the next step without purification.
Synthesis of Compound **34:** Referring to the synthesis of Compound **11,** following purification by prep-HPLC, lyophilization was performed to obtain approximately 248.9 mg of a white solid, with a yield of 55.6%; MS: [M+H]⁺1133.6348.
Synthesis of Compound **35:** Referring to the synthesis of Compound **12,** following the addition of 220 mg of Starting Material **34** and purification by prep-HPLC, lyophilization was performed to obtain approximately 137.3 mg of a white solid, with a yield of 63.4%; MS: [M+H]⁺1115.6147.
Synthesis of Compound **36:** Referring to the synthesis of Compound **22,** 120 mg of Starting Material **35** was added, and the product was directly used in the next step without purification.
Synthesis of Compound **37:** Referring to the synthesis of Compound **23,** following purification by prep-HPLC, the organic phase was spun off and lyophilization was performed to obtain approximately 112.4 mg of a white solid, with a yield of 57.1%; MS: [M+H]⁺1827.9857.

### Synthesis of Compound ama-6

Referring to the synthesis of Compound ama-**1**, following the addition of 20 mg of Compound **37** and preparation and purification, lyophilization was performed to obtain approximately 7 mg of a light-yellow solid, with a yield of 40%. MS: [M+H]⁺1599.8324.

### Example 7: Synthesis of Small Molecule Payload ama-7

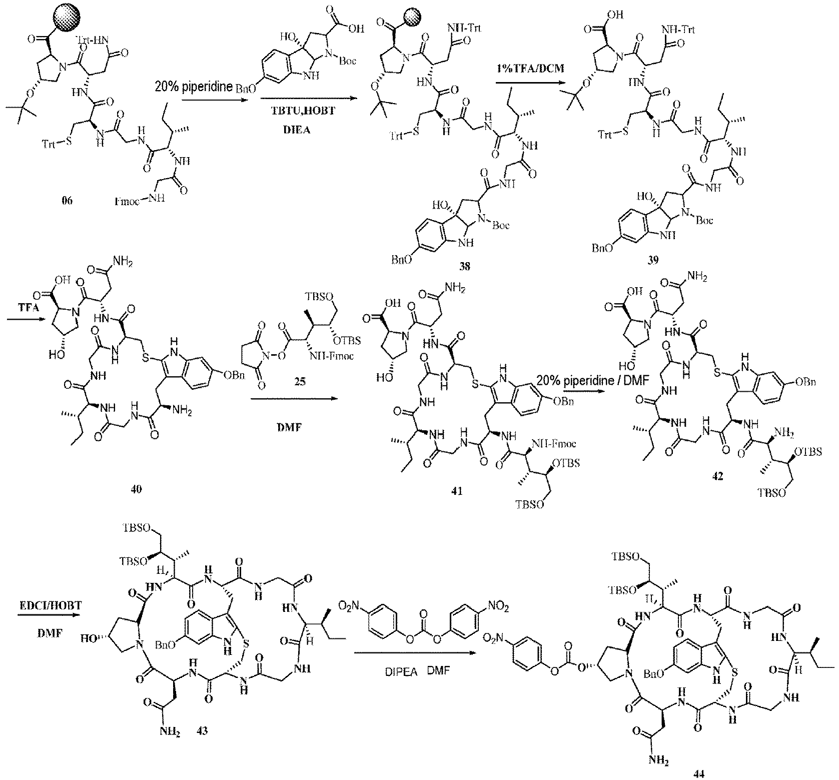

Synthesis of Compound **38:** Referring to the synthesis of Compound **07,** loading was calculated to approximately 0.32 mmol/g.
Synthesis of Compound **39:** Referring to the synthesis of Compound **08,** spin drying was performed to obtain a brown oily substance which was directly used in the next step without purification.
Synthesis of Compound **40:** Referring to the synthesis of Compound **09,** following preparation and purification, lyophilization was performed to obtain approximately 526.4 mg of a light-yellow solid was obtained, with a yield of 34%, as calculated starting from the amount of Compound **38** used. MS: [M+H]⁺866.3512.
Synthesis of Compound **41:** Referring to the synthesis of Compound **10,** 500 mg of Compound **40** was added and target peaks were collected followed by lyophilization to obtain 531.6 mg of an off-white solid, with a yield of 63%. MS: [M+H]⁺1461.6624.
Synthesis of Compound **42:** Referring to the synthesis of Compound **11,** 525 mg of Compound **41** was added, purification was performed and target peaks were collected, followed by lyophilization to obtain 368.5 mg of a light-yellow solid, with a yield of 82.8%. MS: [M+H]⁺1239.5764.
Synthesis of Compound **43:** Referring to the synthesis of Compound **12,** 350 mg of Compound **42** was added, target peaks were collected and lyophilization was performed to obtain 223.4 mg of a light-yellow solid, with a yield of 64.7%. MS: [M+H]⁺1221.5748.
Synthesis of Compound **44:** Referring to the synthesis of Compound **22,** 220 mg of Compound **43** was added, and the product was directly used in the next step without purification. 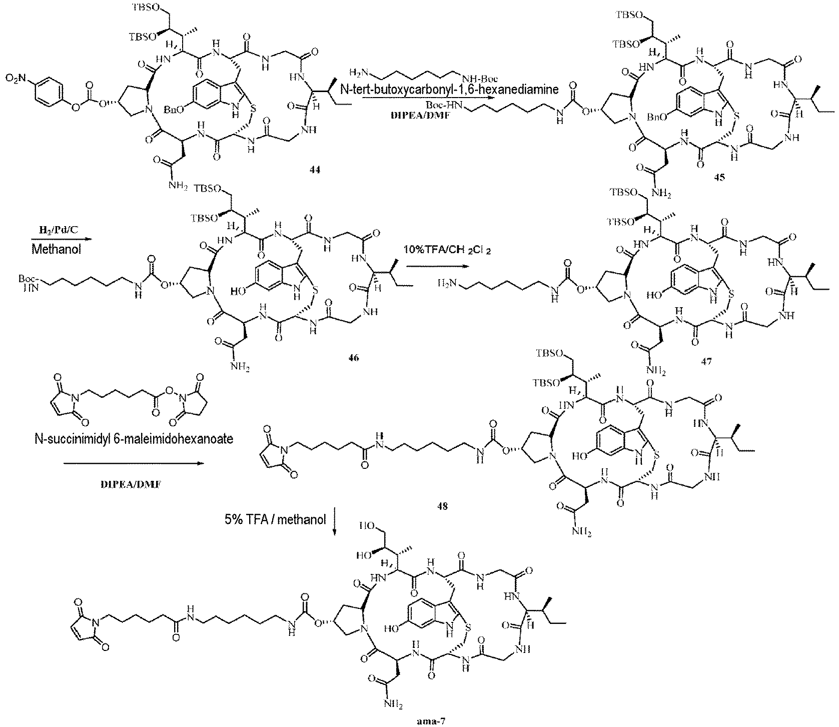
Synthesis of Compound **45:** Referring to the synthesis of Compound **30,** following preparation and purification, lyophilization was performed to obtain approximately 130.2 mg of a light-yellow solid was obtained, with a yield of 49.4%. MS: [M+H]⁺1463.7524.
Synthesis of Compound **46:** Approximately 125 mg of Compound **45** was dissolved in 1 ml of methanol, after which 12.5 mg of 10% Pd/C was added and a hydrogenation reduction reaction was allowed to proceed at 40°C and 0.5 Mpa for 12 hours, with HPLC used to confirm reaction completion, thereafter, the Pd/C was removed via filtration and the filtrate was collected and concentrated to obtain a brown oily material which was fed directly into the next step without purification. Synthesis of Compound **47:** Crude product constituted by Compound **46** was dissolved in 1ml of a 10% TFA dichloromethane solution, after which the reaction was allowed to proceed under stirring and nitrogen gas for one hour at room temperature, HPLC was used to confirm completion of the reaction, the solution was spun off and the resulting product was directly fed into the following step without purification.
Synthesis of Compound **48:** Referring to the synthesis of Compound **32** and calculating based on a 100% yield for the preceding step, following preparation and purification, target peaks were collected and lyophilization was performed to obtain approximately 20.6 mg of a light-yellow solid, with a total three-step yield of 16%. MS: [M+H]⁺1466.7436.

### Synthesis of Compound ama-7

Referring to the synthesis of Compound ama-**1**, following the addition of 10 mg of starting material **48** and preparation and purification, target peaks were collected and lyophilization was performed to obtain approximately 3.2 mg of an off-white solid, with a yield of 37.9%. MS: [M+H]⁺1238.6512.

### Example 8: Synthesis of Small Molecule Payload ama-8

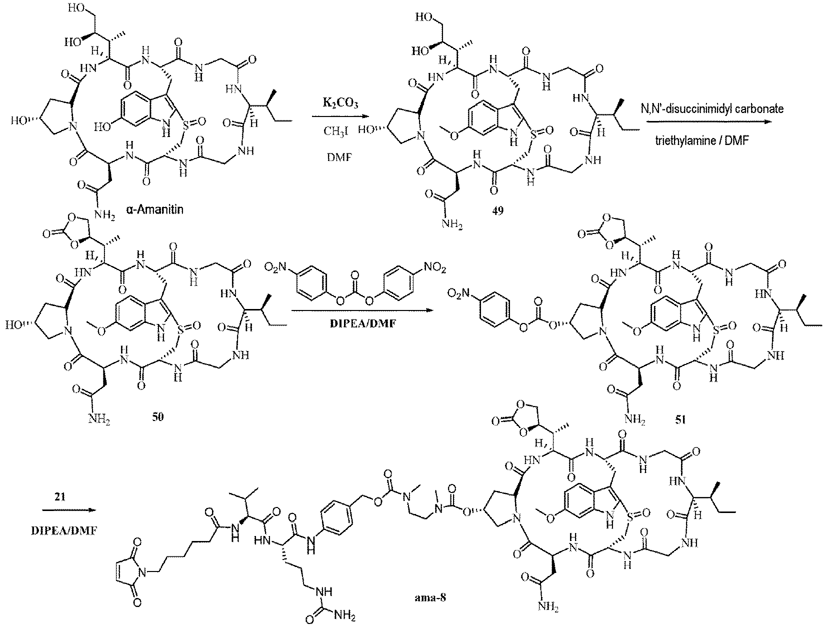

Synthesis of Compound **49:** 30 mg of *α*-amanitin was dissolved with 1 ml of DMF, after which 9 mg of potassium carbonate (3 eq) was added and the mixture was stirred for one hour at room temperature, followed by addition of 13 ul of iodomethane (10 eq) and stirring for 12 hours at room temperature, followed by purification by prep-HPLC, and lyophilization was performed to obtain approximately 9.1 mg of target compound, with a yield of 30%. MS: [M+H]⁺933.4031.
Synthesis of Compound **50:** Compound **49** obtained above was dissolved in 1 ml of DMF, followed by the addition of 25 mg (10 eq) *N,N*'-disuccinimidyl carbonate and the addition of 0.14 ml (20 eq) triethylamine, after which stirring was performed at room temperature for 12 hours, thereafter, once the starting material was completely reacted, purification by prep-HPLC was performed, target peaks were collected and lyophilization was performed to obtain 5.6 mg of a white solid, with a yield of 59.9%; MS: [M+H]⁺959.4501.
Synthesis of Compound **51:** Referring to the synthesis of Compound **22,** all 5.6 mg of Compound **50** obtained above was added to the reaction and the product was directly fed into the next step without purification.

### Synthesis of Compound ama-8:

Referring to the synthesis of Compound **23,** following preparation and purification, lyophilization was performed to obtain approximately 3.2 mg of a white solid, with a yield of 32.8%; MS: [M+H]⁺1671.7211.

### Example 9: Synthesis of Small Molecule Payload ama-9

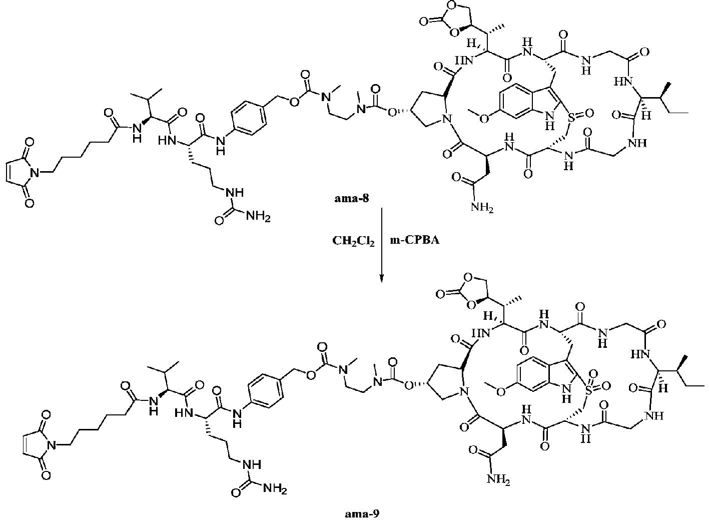

Approximately 2 mg of Compound ama-**8** was dissolved in 0.5 ml of dichloromethane, followed by the addition of 0.6 mg (3 eq) *m*-chloroperoxybenzoic acid (*m*-CPBA), after which the reaction was allowed to proceed under stirring for three hours at room temperature, subsequently, purification by prep-HPLC was performed and target peaks were collected followed by lyophilization to obtain approximately 0.8 mg of a white solid, with a yield of 39.6%; MS: [M+H]⁺1687.7142.

### Example 10: Synthesis of Small Molecule Payload ama-10

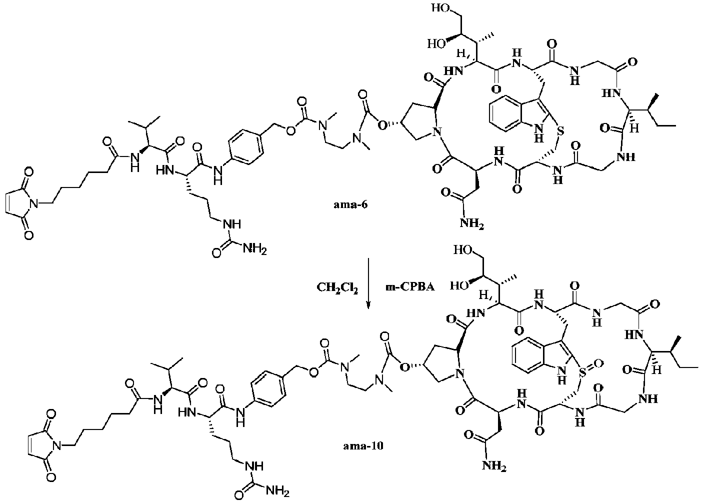

Approximately 4 mg of Compound ama-**6** was dissolved in 0.5 ml of dichloromethane, followed by the addition of 0.5 mg (1.2 eq) *m*-chloroperoxybenzoic acid (*m*-CPBA), after which the reaction was allowed to proceed under stirring for two hours at room temperature, subsequently, purification by prep-HPLC was performed and purified and target peaks were collected followed by lyophilization to obtain approximately 1.2 mg of a white solid, with a yield of 29.7%; MS: [M+H]⁺1615.7320.

### Example 11:

The following compounds were prepared as part of the present example:

| Compound | Structure | Preparation |
|---|---|---|
| 1 | 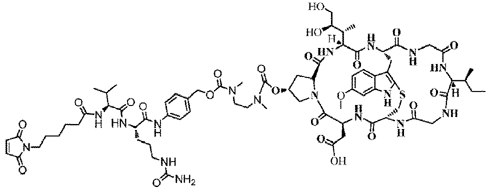 | Refer to ama-1 |
| 2 | 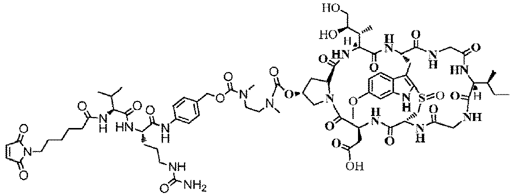 | Refer to ama-2 |
| 3 | 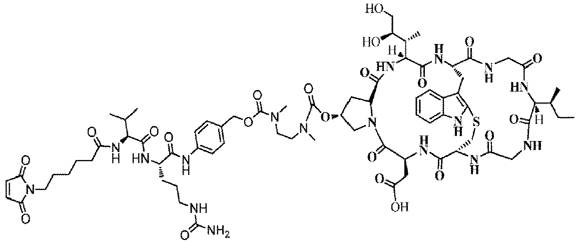 | Refer to ama-1 |
| 4 | 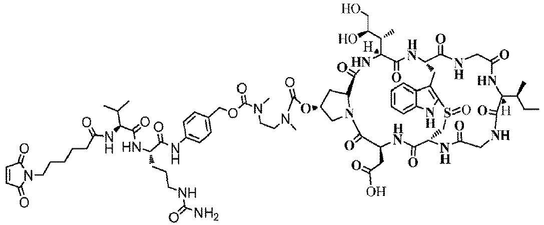 | Refer to ama-2 |
| 5 | 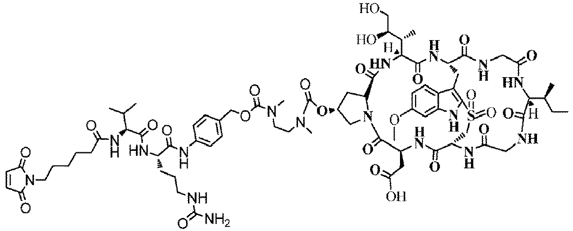 | Refer to ama-9 |
| 6 | 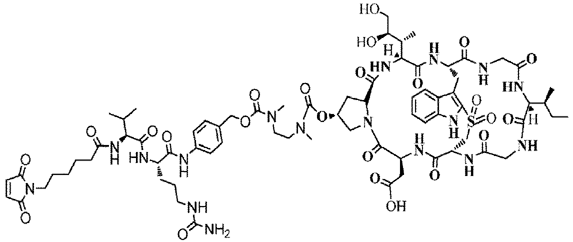 | Refer to ama-9 |
| 7 | 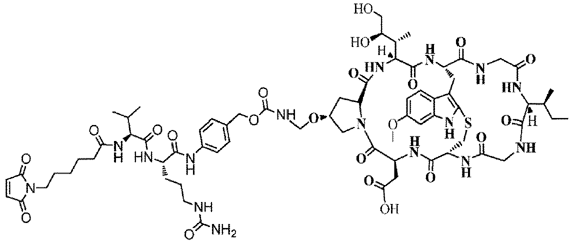 | Refer to ama-1 |
| 8 | 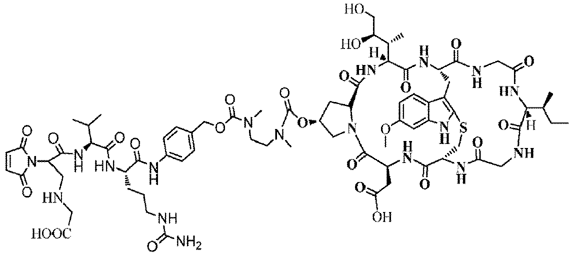 | Refer to ama-1 |
| 9 | 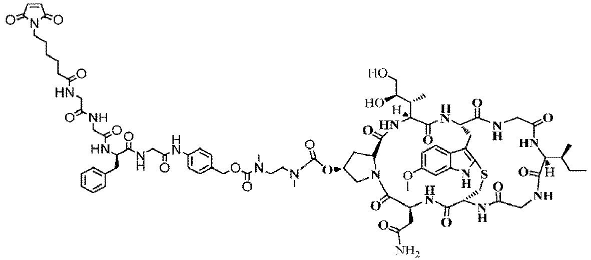 | Refer to ama-1 |
| 10 | 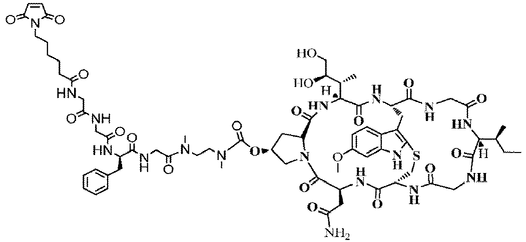 | Refer to ama-1 |
| 11 | 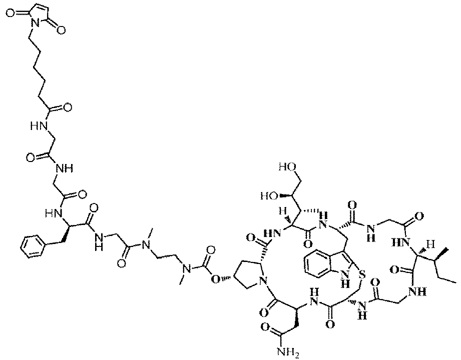 | Refer to ama-1 |
| 12 | 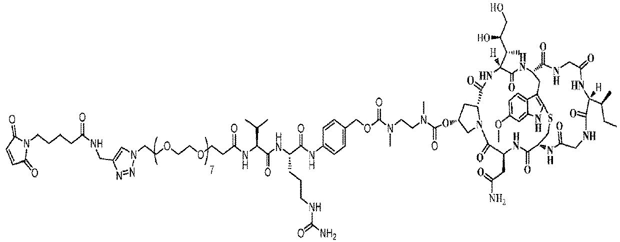 | Refer to ama-1 |
| 13 | 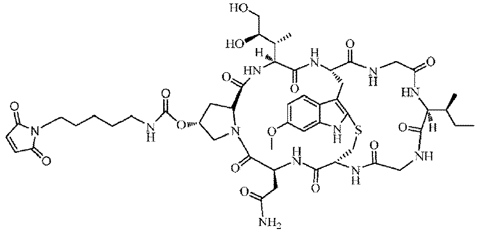 | Refer to ama-5 |
| 14 | 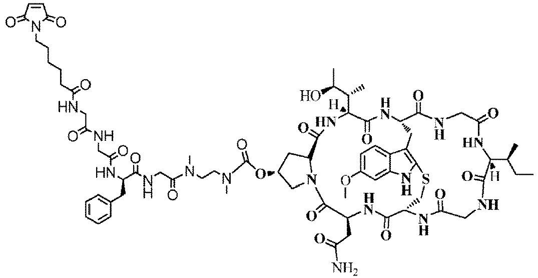 | Refer to ama-1 |

Example 12: Preparation of Antibody-Drug Conjugate 1) General Conjugation Method: Following preliminary purification, antibody molecules with a monomer ratio greater than 95% were exchanged into phosphate buffer containing EDTA at a concentration of 10 mg/ml using an ultrafiltration centrifuge tube. TCEP in a molar amount 10-fold that of the antibody was added and the resulting reaction was allowed to proceed for two hours. Using an ultrafiltration centrifuge tube to exchange the solution into pH 6.5 phosphate buffer, DHAA in a molar amount 10-fold that of the antibody was thereafter added and the resulting reaction was allowed to proceed for two hours. Next, payload in a molar amount 3-fold that of the antibody was added and the resulting reaction was allowed to proceed for four hours. Once the reaction was finished, an ultrafiltration centrifuge tube with a molecular weight cut-off of 30 KDa was used to exchange the solution into PBS and the uncoupled payload was removed.

### 2) Antibody-Drug Conjugate DAR Assay

Monomer Ratio Assay Conditions:
Samples were centrifuged at 14,000 rpm for five minutes and the supernatant was taken for analysis.

Instrument: Waters e2695 (2489UV/Vis);
Chromatography column: TSKgel G3000SWXL (7.8 × 300 mm, 5 µm) ;
Mobile phase: A: 50 mM PB, 300 mM NaCl, 200 mM Arg, 5% IPA, pH 6.5;
Mobile Phase A was eluted isocratically for 30 minutes; flow rate: 0.714 ml/min, column temperature: 25°C, detection wavelength: 280 nm.

DAR Assay Conditions:
Samples were centrifuged at 14,000 rpm for five minutes and the supernatant was taken for analysis.

Instrument: Waters H-class (TUV);
Chromatography column: Proteomix HIC Butyl-NP5 (4.6 × 35 mm, 5 µm) ;
Mobile phase: A: 1.5M ammonium sulfate, 0.025 M anhydrous sodium phosphate, pH 7.0
B: 0.025 M anhydrous sodium phosphate, 25% IPA, pH 7.0

Mobile Phase A was used to equilibrate the chromatographic column, after which Mobile Phases A and B were gradient eluted, with a flow rate of 0.8 ml/min; the column temperature was 25°C, and the detection wavelength was 214 nm.

The structural formula of A0301 is as follows:

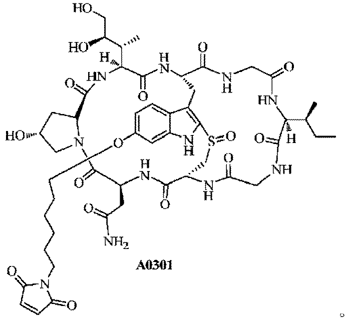

### 3) Results

Table 1 shows that the antibody conjugate linked from the hydroxyproline site exhibits a relatively favorable monomer rate and a relatively high DAR. Due to the similarity of their structures, Compounds **1** through **14** also exhibit a relatively favorable monomer rate and a relatively high DAR.

**Table 1**

| Number | Monomer Ratio | DAR |
|---|---|---|
| Herceptin-007 | 93.86% | - |
| Herceptin-007-A0301 | 93.47% | 1.81 |
| Herceptin-007-ama-3 | 95.08% | 1.87 |
| Herceptin-007-ama-4 | 96.34% | 1.86 |
| Herceptin-007-ama-6 | 95.44% | 1.7 |
| Herceptin-007-ama-8 | 95.27% | 1.86 |
| Herceptin-007-ama-9 | 95.20% | 1.83 |
| Herceptin-007-ama-10 | 95.61% | 1.86 |

Example 13: Activity Testing *in Vitro*

### 1) Experimental Materials

Cells: Obtained from the Cell Bank of the Chinese Academy of Sciences
Tumor cell culture medium: Gibco
FBS: BIOWEST

### 2) Culture Medium Preparation

Growth medium (with 10% FBS, penicillin/streptomycin (100 U/ml)
Detection medium (with 1% FBS, penicillin/streptomycin (100U/ml)

### 3) Procedure

The biosafety cabinet UV lamp was turned on 30 min in advance to carry out irradiation and air exchange was performed for three minutes thereafter. The growth medium, detection medium, D-PBS and trypsin were preheated in a 37°C thermostatic water bath, after which they were placed in a biosafety cabinet following surface sterilization. Cells with a confluence rate of approximately 80% were placed in the biosafety cabinet, old medium was aspirated off, and the cells were washed with D-PBS, followed by aspiration and digestion with trypsin for 2 to 3 minutes, after which growth medium was added to neutralize the reaction and centrifugation was performed at 1,200 rpm for three minutes. The supernatant obtained following centrifugation was aspirated off and 4 ml of assay medium was mixed in evenly, after which 100 ul of solution was taken for counting (wherein, 50 ul of cell solution was mixed well with 50 ul of Trypan Blue Stain, and counting was thereafter performed) . Cells were plated based on a pre-set number of cells with 80 ul per well plated in 96-well plates; Wells E11, F11 and G11 were filled with only 80 ul of detection medium and 150 ul of DPBS was added to the edge wells. Dilution of Antibody Solution: 300 ul of test product solution with an initial concentration of 5 uM was prepared in the first column of a V-type 96-well plate, with 210 ul of detection medium added to each of Columns 2 through 10; 30 ul was then taken from the pre-mixed first column and added to the second column, mixed up and down 10 times using a pipette and the pipette tip was thereafter discarded; the subsequent seven concentrations were then prepared sequentially using the same procedure. Twenty-four hours after plating, diluted antibody was added at 20 ul per well with an additional control established; only 20 ul of detection medium was added to Column 11, two duplicate wells were established for each concentration and cells were vortexed at 550 rpm for three minutes following addition.

### 4) Assay

After four days, the MTS reagent was removed and samples were thawed at room temperature while ensuring no light exposure and thorough vortexing was performed; 20 µL of CellTiter 96® One Solution Reagen MTS reagent was added for every 100 µL of cell culture volume along the side wall of the wells, and the plate surface was gently tapped to mix the MTS solution evenly, after which the samples were placed in a cell culture incubator for static incubation for two hours while ensuring no light exposure occurred. After the reaction was finished, the 96-well plate was taken out, OD490 nm absorbance values were assayed using a microplate reader, and corresponding data was recorded, sorted, and stored.

### 5) Results

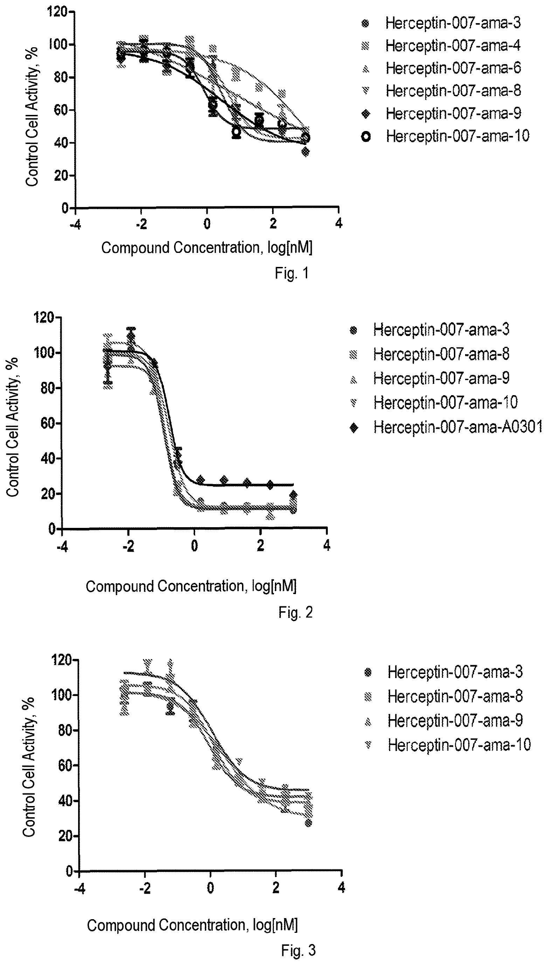

As shown in Table 2 and Figures 1 through 3, good anti-tumor activity was demonstrated in the cell lines BT474/SKBR3/N87. Due to the similarity of their structures, Compounds **1** through **14** also exhibit relatively favorable anti-tumor activity.

**Table 2**

| Number | BT474 IC50 (nM) | SKBR3 IC50 (nM) | N87 IC50 (nM) |
|---|---|---|---|
| Herceptin-007-a ma-3 | 19.31 | 0.21 | 11.72 |
| Herceptin-007-ama-4 | 200∼1000 | > 1000 | > 1000 |
| Herceptin-007 -ama-6 | 200∼1000 | >1000 | ∼1000 |
| Herceptin-007-ama-8 | 9.87 | 0.15 | 6.61 |
| Herceptin-007-ama-9 | 34.77 | 0.14 | 5.92 |
| Herceptin-007-ama-10 | 7.36 | 0.14 | 24.19 |
| Herceptin-007-A0301 | > 1000 | 0.25 | >1000 |

In the preceding section, embodiments of the present invention were described in detail in conjunction with examples in order to fully elucidate how the present invention uses certain technical means to solve corresponding technical problems and achieve technical efficacy, and these may be used as a basis for implementation. It should be noted that individual embodiments of the present invention and the individual features of each embodiment can be combined with each other and the resulting technical solutions shall remain within the scope of the present invention, provided there are no conflicts.

## Claims

1. A toxin conjugate as claimed in Structural Formula (I) or a pharmaceutically acceptable salt thereof: 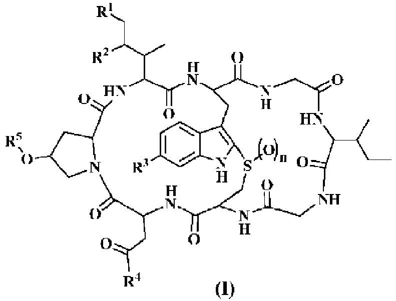 wherein: n = 0, 1 or 2, R¹ corresponds to -H or -OH, R² corresponds to -H or -OH, R³ corresponds to -H, -OH or C₁₋₆ alkyl, R⁴ corresponds to -NH₂ or -OH, R⁵ corresponds to -L-A, A corresponds to the biological macromolecule moeity that binds to a target, and L corresponds to any chemical structure connecting an amanitin derivative and a biological macromolecule.

2. The toxin conjugate as claimed in claim 1 or a pharmaceutically acceptable salt thereof, which is **characterized in that** the chemical structure of L includes a cleavable or non-cleavable structure.

3. The toxin conjugate as claimed in claim 1 or a pharmaceutically acceptable salt thereof, which is **characterized in that** A includes an antibody or antigen-binding fragment or antigen-binding polypeptide thereof.

4. The toxin conjugate as claimed in claim 1 or a pharmaceutically acceptable salt thereof, which is **characterized in that** the conjugation site of L and the target-binding biomolecule A includes the following structure:

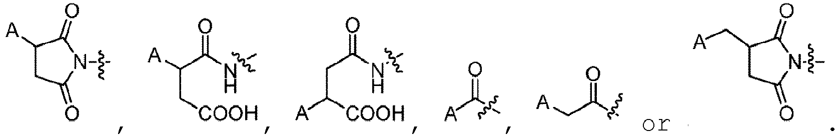

5. The toxin conjugate as claimed in claim 1 or a pharmaceutically acceptable salt thereof, which is **characterized in that** L is connected to the toxin via an ester or ether.

6. A drug compound which includes a toxin conjugate as claimed in claims 1 through 5 or a pharmaceutically acceptable salt thereof.

7. A use of a toxin conjugate as claimed in claims 1 through 5 or a pharmaceutically acceptable salt thereof in the preparation of an anti-tumor drug or anti-cancer drug.

8. The use as claimed in claim 7, which is **characterized in that** said antitumor drug or anti-cancer drug corresponds to an anti-lung cancer drug, an anti-kidney cancer drug, an anti-urinary tract cancer drug, anti-colorectal cancer drug, anti-prostate cancer drug, anti-glioblastoma drug, anti-ovarian cancer drug, anti-pancreatic cancer drug, anti-breast cancer drug, anti-melanoma drug, anti-liver cancer drug, anti-bladder cancer drug, anti-malignant lymphoma drug, anti-leukemia drug, anti-gastric cancer drug or an anti-esophageal cancer drug.
